# EUROPEAN PATENT APPLICATION

(11) **EP 4 792 772 A1**
(43) Date of publication of application: **19.08.2026**
(21) Application number: 24877488.7
(22) Date of filing: 08.10.2024
(51) Int. Cl.: A61C 7/00, A61C 7/08, A61C 9/00, G06T 1/00, G06T 17/00, G16H 30/00, G16H 50/50, G06T 19/20, B33Y 80/00, B33Y 50/00

(54) **METHOD FOR DESIGNING ORTHODONTIC DEVICE ACCORDING TO PARTIAL DESIGNATION OF TEETH**

(30) Priority: 12.10.2023 KR 20230136283
(71) Applicant: ODS Co., Ltd, Incheon 21990 (KR)
(72) Inventor: SIM, Miyoung, Incheon 21986 (KR); PARK, Sungwon, Incheon 21986 (KR)
(74) Representative: You Patent
(86) International application number: PCT/KR2024/015249
(87) International publication number: WO 2025/079951

(57) **Abstract**

The present invention relates to a method for designing an orthodontic appliance according to partial designation of a tooth using a computer. The method includes: displaying a patient's dentition (S01); setting up all teeth in the dentition and displaying the dentition in a state after orthodontic correction (S02); designating a tooth in the dentition (S03); designating a specific portion of the designated tooth (S04); and inputting a thickness of an orthodontic appliance that contacts the designated specific portion of the designated tooth (S05).

## Description

### Technical Field

The present invention relates to a method for designing an orthodontic appliance that is output (formed) by a 3D printer using a computer.

More particularly, the invention relates to a technique in which digital information obtained by scanning a patient's oral condition using a 3D scanner is displayed on a screen, and, in a state in which the dentition is set up according to an orthodontic objective, characteristics of individual teeth are reflected so that a precise orthodontic appliance is designed through partial designation of the teeth.

### Background Art

A conventional method for manufacturing an orthodontic appliance is as follows.

First, a practitioner examines a patient's oral structure and then prepares a tooth model having the same shape as the patient's teeth. The tooth model of the patient is generally produced by first making an impression of the patient's oral structure and then forming the model using plaster or the like.

With respect to the plaster tooth model thus prepared, a sheet-type polyol material is pressed at a high temperature in an upper and lower direction by a molding machine, thereby producing a transparent orthodontic appliance suitable for the patient. This is a conventional technique.

Recently, when 3D scanning data regarding a patient's tooth structure is transmitted to a dental laboratory, the dental laboratory produces a tooth model based on the received information and manufactures a transparent orthodontic appliance using a pressing method. Such techniques have been widely used.

An advanced technique for manufacturing an orthodontic appliance using a 3D printer has been disclosed in Korean Patent No. 10-2500642.

The above patent technology relates to a method in which a patient's oral condition is displayed, teeth to be orthodontically corrected are selected, and a thickness value and a spacing value of the orthodontic appliance for achieving the orthodontic objective are input.

However, the above patent technology has a technical feature in that the orthodontic appliance is partially designed only for teeth to be corrected and in contact therewith. Accordingly, it does not consider teeth that are not subject to orthodontic correction, and has a limitation in that partial differences within a single tooth cannot be reflected in the orthodontic appliance.

### Specific Instruction for Implementing the Invention

### Technical Problem

A method for manufacturing a transparent orthodontic appliance using a 3D printer according to the present invention has as a technical objective to significantly reduce the time and labor required for manufacturing an orthodontic appliance by directly outputting the orthodontic appliance using a 3D printer without producing a separate tooth model, thereby improving the convenience and economic efficiency of manufacturing the orthodontic appliance.

Another technical objective of the present invention is to provide a design of an orthodontic appliance capable of securing a more precise orthodontic force through precise design of portions of the orthodontic appliance that contact not only teeth to be orthodontically corrected but also teeth that are not subject to orthodontic correction.

A further technical objective of the present invention is to provide a more precise and efficient design of an orthodontic appliance by reflecting partial characteristics of teeth in the design of the orthodontic appliance.

### Technical Solution

In order to achieve the above technical objectives, the present invention provides:
a step of displaying a patient's dentition (S01);
a step of setting up all teeth in the dentition and displaying the dentition in a state after orthodontic correction (S02);
a step of designating a tooth in the dentition (S03);
a step of designating a specific portion of the designated tooth (S04);
a step of setting a thickness of an orthodontic appliance that contacts the designated portion of the designated tooth (S05); and
a step of setting a gap between the orthodontic appliance and the tooth at the designated portion of the designated tooth (S06).

In order to achieve the above technical objectives,
the present invention further provides that the step of designating a specific tooth (S03) may designate a single tooth or may designate a plurality of adjacent teeth simultaneously.

### Effects of the Invention

According to the method for designing an orthodontic appliance according to partial designation of a tooth according to the present invention, the orthodontic appliance is designed and manufactured using a computer and a 3D printer, thereby improving the convenience of manufacturing and also enhancing the economic efficiency of the manufacturing process.

According to the method for designing an orthodontic appliance according to partial designation of a tooth according to the present invention, orthodontic force and precision of the orthodontic appliance can be improved by reflecting not only teeth to be orthodontically corrected but also teeth that are not subject to orthodontic correction in the design of the orthodontic appliance.

According to the method for designing an orthodontic appliance according to partial designation of a tooth according to the present invention, orthodontic force and precision of the orthodontic appliance can be improved by partially and differently considering teeth in the design of the orthodontic appliance.

### Brief Description of the Drawings

FIG. 1 illustrates a state in which a tooth is designated according to the present invention.
FIG. 2 illustrates a state in which a portion of a tooth is designated according to the present invention.
FIG. 3 illustrates a step of setting a thickness and a gap of an orthodontic appliance according to the present invention.
FIG. 4 illustrates one embodiment of an orthodontic appliance designed according to the present invention.

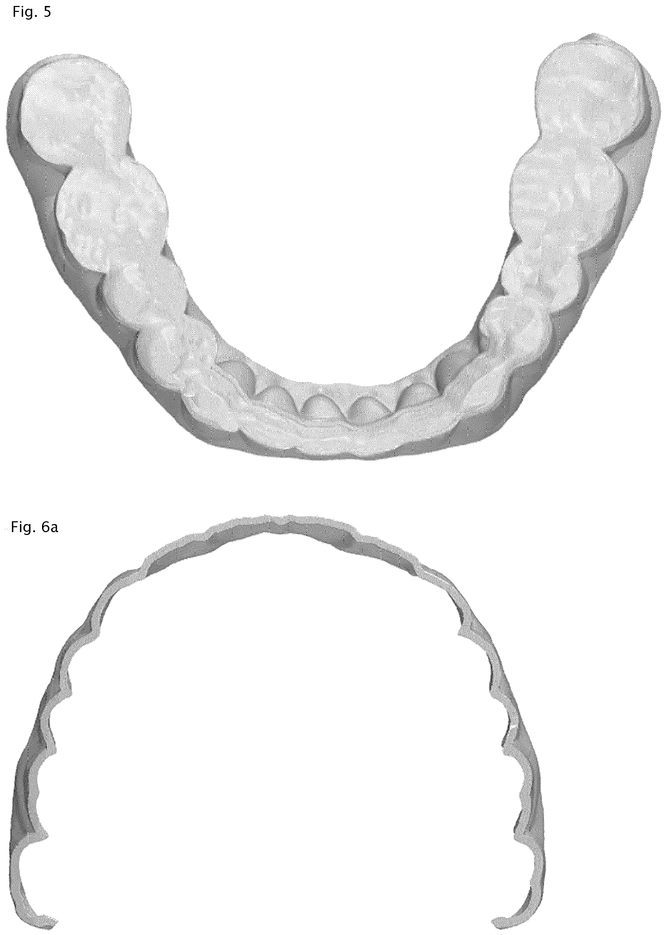

FIG. 5 illustrates another embodiment of an orthodontic appliance designed according to the present invention.

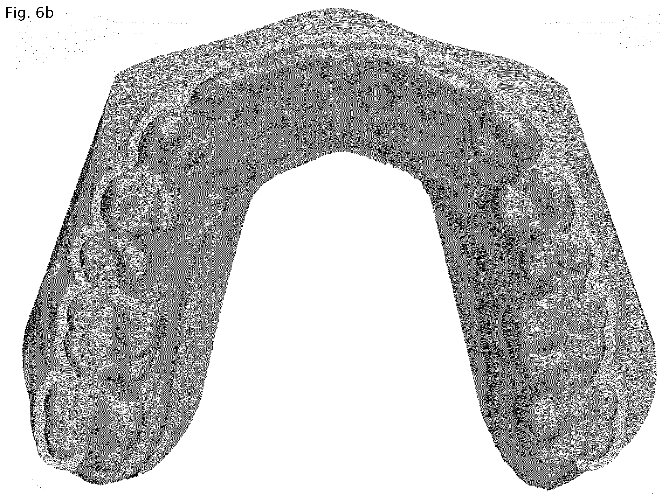

FIGS. 6A and 6B illustrate another embodiment of an orthodontic appliance designed according to the present invention.

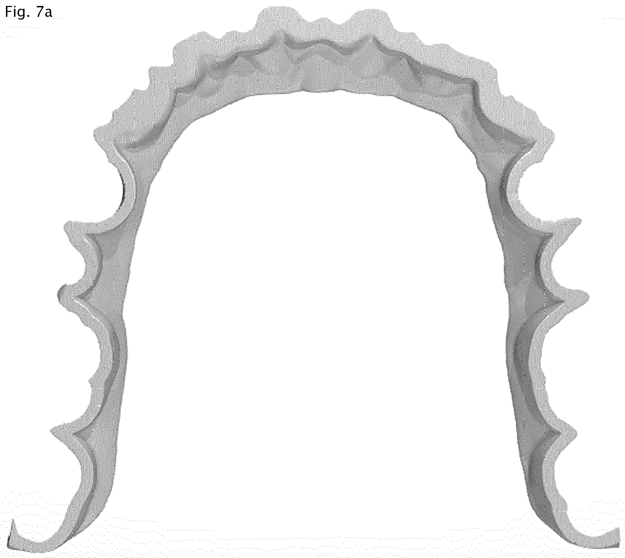

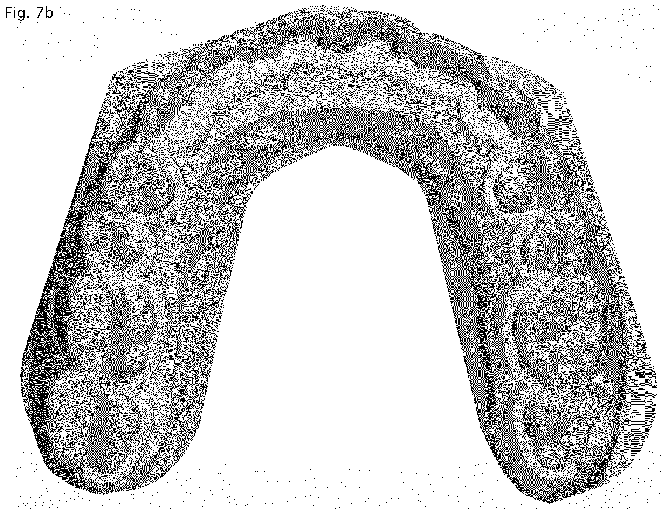

FIGS. 7A and 7B illustrate another embodiment of an orthodontic appliance designed according to the present invention.

### Best Mode for Implementing the Invention

The present invention includes:
a step of displaying a patient's dentition (S01);
a step of setting up all teeth in the dentition and displaying the dentition in a state after orthodontic correction (S02);
a step of designating a tooth in the dentition (S03);
a step of designating a specific portion of the designated tooth (S04);
a step of setting a thickness of an orthodontic appliance that contacts the designated portion of the designated tooth (S05); and
a step of setting a gap between the orthodontic appliance and the tooth at the designated portion of the designated tooth (S06).

### Mode for Implementing the Invention

Hereinafter, a method for manufacturing a transparent orthodontic appliance using a 3D printer according to the present invention will be described in detail step by step.

The technical terms used in the present application are used merely for the purpose of describing the embodiments below and are not intended to limit the scope of the present invention through such technical terms. Unless otherwise specifically defined in the present invention, the technical terms should be understood as conveying meanings generally understood and used by those skilled in the art.

The present invention relates to a method (program) for designing an orthodontic appliance using a computer. Here, the computer includes various devices capable of performing computational processing and providing results to a user, and generally includes an input unit, a processing unit, and an output unit.

In the present specification, an image relating to a tooth model refers to a multidimensional image such as a two-dimensional or three-dimensional image showing the overall arrangement of teeth, and includes images obtained by medical imaging techniques using tomographic imaging. For example, the images may include CT (computed tomography) images, nuclear magnetic resonance computed tomography images (NMR-CT), positron emission tomography (PET) images, cone beam computed tomography (CBCT) images, and images obtained by an oral scanner, among various types of imaging modalities.

In the present specification, the term "tooth to be orthodontically corrected" refers to a tooth whose position is inappropriate or abnormally rotated, and which requires movement of position and/or rotation through orthodontic treatment.

Hereinafter, embodiments of the present invention will be described in detail with reference to the accompanying drawings.

Step S01 is a step of displaying a patient's dentition, and step S02 is a step of setting up all teeth in the dentition and displaying the dentition in a state after orthodontic correction.

The interior of a patient's oral cavity is scanned using a 3D scanner, and the patient's dentition information thus obtained is stored in a computer. An operator retrieves the patient's dentition information stored in the computer and displays it on an image display device (such as a monitor) (step S01). The operator selects teeth that are subject to orthodontic correction from the displayed teeth and performs a process (set-up) of modifying the selected teeth into a corrected state. In this manner, a corrected dentition image is completed and displayed on the screen (step S02).

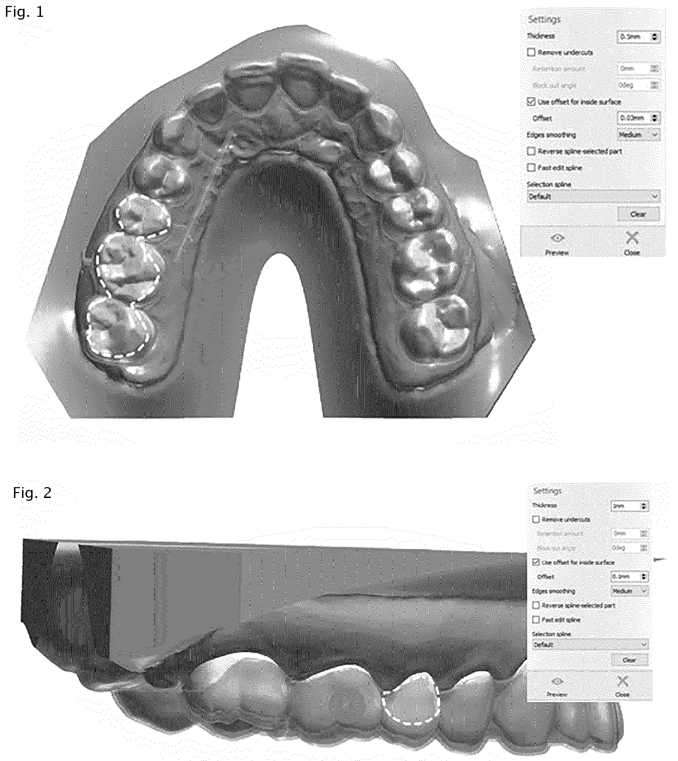

Step S03 is a step of designating a tooth in the dentition. FIG. 1 illustrates a state in which a tooth is designated according to the present invention.

Human dentition consists of approximately 28 to 32 teeth. Unique identification numbers for these teeth are stored in advance in the computer. When the operator inputs or selects a particular number among the teeth, the tooth corresponding to that number is designated. Once a tooth is designated, the designated tooth is displayed in a manner distinguishable from other teeth, for example by changing its color. In step S03, the designation of a tooth may also be performed by directly selecting the tooth using a cursor movement device such as a mouse.

The step of designating a particular tooth (step S03) may designate a single tooth or may designate a plurality of adjacent teeth simultaneously. Even when multiple teeth are designated simultaneously, the designation method may be the same as described above.

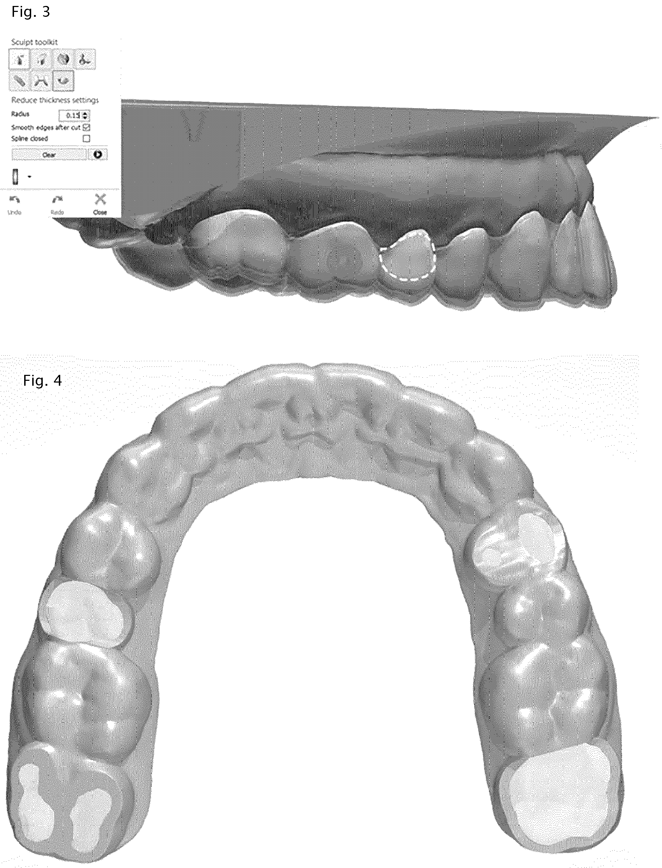

FIG. 2 illustrates a state in which a portion of a tooth is designated according to the present invention, and FIG. 3 illustrates a step of setting the thickness and gap of the orthodontic appliance according to the present invention.

Step S04 is a step of designating a specific portion of the tooth designated in step S03.

Step S04 includes designating one or more portions among an upper surface and multiple side surfaces of each tooth. Each human tooth has a different shape, and the upper surface and side surfaces of each tooth also have unique shapes. Considering this aspect, the present invention includes a method of varying the thickness of the orthodontic appliance for each portion of a tooth.

An orthodontic appliance mounted on teeth closely contacts multiple surfaces of the teeth (such as lingual surfaces, buccal surfaces, labial surfaces, mesial surfaces, distal surfaces, and the like). A technical feature of the present invention is that the thickness of a portion of the orthodontic appliance contacting a tooth is varied according to characteristics of each surface of the tooth. In other words, the thickness of the portion of the orthodontic appliance contacting a specific tooth is not uniformly set to be the same but is varied according to the respective surfaces of the tooth.

The thickness of the orthodontic appliance for each surface of a tooth is determined in consideration of various factors such as convenience in use of the orthodontic appliance and securing effective orthodontic force. Taking such factors into account, the operator designates a specific portion of the target tooth.

A method of designating a specific portion of a tooth may be performed directly by the operator using a cursor movement device such as a mouse. Alternatively, unique identification numbers for each tooth and portions thereof may be stored in advance so that the designation can be easily performed by inputting the identification numbers. In addition to the methods described above, various modifications of designation methods may be adopted, and such modifications should be regarded as simple design changes that fall within the technical scope of the present invention.

Meanwhile, the present invention also includes designating a plurality of adjacent teeth simultaneously and designating side surfaces in a specific direction for the designated teeth. For example, a plurality of adjacent teeth of the maxilla or mandible (including the case of designating all teeth) may be designated, and the lingual surface (the direction toward the tongue) or the buccal surface (the direction toward the cheek) of the designated teeth may be designated. Alternatively, the palatal surface (the surface toward the palate) of the designated teeth may be designated. The thickness of the orthodontic appliance contacting the lingual surface, buccal surface, or palatal surface of the designated teeth may be increased. An orthodontic appliance formed in this manner may be applied to orthodontic correction for arch expansion or arch constriction. For correction of arch constriction, the orthodontic appliance on the buccal surface may be made thicker, whereas for correction of arch expansion, the orthodontic appliance on the lingual surface and/or the palatal surface may be made thicker.

For correction of arch expansion or arch constriction, it is also possible to form the lingual surface, buccal surface, or palatal surface of the orthodontic appliance without curvature. In other words, an entire side surface of the orthodontic appliance may be smoothly formed with a predetermined sufficient thickness without following the shape of the dentition. In such a case, a greater orthodontic force may be applied overall to the dentition.

Meanwhile, in order to correct arch expansion or arch constriction, it is possible to form a transverse reinforcing member on the surface of the orthodontic appliance where the orthodontic force is applied. The reinforcing member is configured to exert and maintain a stronger orthodontic force. The reinforcing member may be provided on portions of the lingual surface, buccal surface, or palatal surface of the orthodontic appliance where additional orthodontic force is required. The reinforcing member may be in the form of a simple beam or an H-beam. However, the cross-sectional shape of the reinforcing member may be modified in various ways, such as circular or triangular shapes. When the orthodontic appliance is printed with the reinforcing member designed therein, the orthodontic appliance will be formed with a protruding thickened portion corresponding to the shape of the reinforcing member at the portion where the reinforcing member is formed.

Step S05 is a step of setting a thickness of the orthodontic appliance that contacts the portion designated in step S04 of the tooth designated in step S03.

A feature of the present invention is that the thickness of the orthodontic appliance may be partially varied with respect to a single tooth. After the steps of designating a tooth and designating a portion of the tooth are completed, the operator performs a process of setting the thickness of the orthodontic appliance for the designated portion. As described above, the thickness of the orthodontic appliance is determined in consideration of various factors such as convenience in use of the orthodontic appliance and securing effective orthodontic force.

When the relevant portion of the tooth is designated, an input window (pop-up menu) is automatically displayed, and the operator may set the thickness value by inputting a specific numerical value into the input window or by gradually adjusting the value by pressing (+)/(-) buttons (for example, setting the increment of one button to 0.05 mm).

The method for designing an orthodontic appliance according to the present invention includes not only varying the thickness of the orthodontic appliance for each tooth but also partially varying the thickness of the orthodontic appliance for a particular tooth.

For example, the operator may design the orthodontic appliance such that portions of the orthodontic appliance contacting anterior teeth (incisors) and molars have different thicknesses, thereby allowing the orthodontic force of the orthodontic appliance according to the present invention to be effectively distributed and applied.

In the case of teeth for which securing orthodontic force is particularly required, it is preferable that the thickness value of the portion of the orthodontic appliance contacting the tooth be set to a relatively large value.

In addition, in the step of setting the thickness of the orthodontic appliance (step S05), the thickness may be set to zero so that no orthodontic appliance is formed at the designated portion.

When the orthodontic appliance is formed on an occlusal surface (i.e., the upper or chewing surface of a molar), it may be effective in cases where orthodontic correction through tooth intrusion is required, because intrusion correction may be achieved by the mastication action of the tooth.

However, if an orthodontic appliance is formed on the occlusal surface when intrusion correction is not required, such a portion would not only be unnecessary for orthodontic treatment but would also interfere with the patient's mastication and may adversely affect the temporomandibular joint. If the orthodontic appliance is unnecessary on the occlusal surface, the thickness of the orthodontic appliance corresponding to the occlusal surface of the tooth may be set to zero, so that no orthodontic appliance is formed in that portion. Meanwhile, the thickness of the orthodontic appliance may be minimized within a range that does not affect orthodontic force, thereby reducing discomfort to the patient when wearing the orthodontic appliance.

**Step S06** is a step of designating a gap between the orthodontic appliance and the tooth at the portion designated in step S04 of the tooth designated in step S03.

A technical feature of the present invention is that the gap between the tooth and the orthodontic appliance may be set not only for each tooth but also partially for each tooth. For example, when orthodontic correction requires pushing a molar or premolar toward the buccal side (the direction toward the cheek), the gap on the lingual side (the direction toward the tongue) should be minimized so that the orthodontic appliance is closely fitted. On the other hand, on the buccal side, the thickness of the orthodontic appliance may be minimized or set to zero, and it is preferable that a predetermined gap be formed between the orthodontic appliance and the tooth. Conversely, when orthodontic correction requires pushing the tooth toward the lingual side, the design may be implemented in a manner opposite to the above description. Meanwhile, when orthodontic correction requires extrusion of a tooth, the gap on a tooth surface (such as the occlusal surface or an incisal surface) of the tooth to be extruded may be formed relatively larger so that extrusion of the tooth can be more easily achieved.

Such modified embodiments as described above should also be regarded as falling within the technical scope of the present invention.

Meanwhile, when it is advantageous for orthodontic treatment that the orthodontic appliance penetrate as deeply as possible into a specific interdental space (i.e., a space between adjacent teeth), it is preferable that the gap between the orthodontic appliance and the side surfaces of teeth adjacent to the interdental space be minimized. Furthermore, when orthodontic force is particularly required, it is preferable that the thickness of the orthodontic appliance be partially increased.

The method of designating the gap between the orthodontic appliance and the tooth may be implemented in the same manner as described above with respect to setting the thickness value, in that when a relevant portion is designated, an input window (pop-up menu) is automatically displayed and the operator may input a specific numerical value or gradually adjust the value by pressing (+)/(-) buttons.

The description of the configuration and effects described above relates to one embodiment of the present invention and is not intended to limit the scope of the claims of the present invention. Various changes and modifications may be made without departing from the technical spirit of the present invention, and such simple design modifications will be apparent to those skilled in the art and should be regarded as falling within the technical scope of the present invention.

## Claims

1. A method for designing an orthodontic appliance using a computer, the method
comprising:
displaying a patient's dentition (S01);
setting up all teeth in the dentition and displaying the dentition in a state after orthodontic correction (S02);
designating a tooth in the dentition (S03);
designating a specific portion of the designated tooth (S04); and
setting a thickness of an orthodontic appliance that contacts the designated portion of the designated tooth (S05),
wherein the method is a method for designing an orthodontic appliance according to partial designation of a tooth using a computer.

2. The method of claim 1,
wherein step S03 is performed first and step S02 is subsequently performed.

3. The method of claim 1 or claim 2, further comprising:
setting a gap between the orthodontic appliance and the tooth at the designated portion of the designated tooth (S06).

4. The method of claim 1 or claim 2,
wherein the step of designating a specific tooth (S03) includes designating a single tooth or designating a plurality of adjacent teeth simultaneously.

5. The method of claim 1 or claim 2,
wherein the step of designating a specific portion of the designated tooth (S04) includes designating one or more among an upper surface and a plurality of side surfaces of each tooth.

6. The method of claim 1 or claim 2,
wherein the step of setting the thickness of the orthodontic appliance that contacts the designated portion of the designated tooth (S05) includes setting the thickness to zero such that the orthodontic appliance is not formed on the designated portion.

7. The method of claim I or claim 2,
wherein step S03 includes designating a plurality of adjacent teeth in a maxilla or a mandible or designating all teeth,
step S04 includes designating at least one of a lingual surface, a buccal surface, and a palatal surface of the designated teeth, and
step S05 includes setting the thickness of a portion of the orthodontic appliance contacting the surface designated in step S04 to be greater than that of other portions.

8. The method of claim 1 or claim 2,
wherein step S03 includes designating a plurality of adjacent teeth in a maxilla or a mandible or designating all teeth,
step S04 includes designating at least one of a lingual surface, a buccal surface, and a palatal surface of the designated teeth, and
the method further comprises forming the portion of the orthodontic appliance contacting the surface designated in step S04 to be smoothly formed without curvature while having a predetermined thickness (S07).

9. The method of claim I or claim 2,
wherein step S03 includes designating a plurality of adjacent teeth in a maxilla or a mandible or designating all teeth,
step S04 includes designating at least one of a lingual surface, a buccal surface, and a palatal surface of the designated teeth, and
the method further comprises forming a reinforcing member on the surface designated in step S04 in order to increase orthodontic force (S08).
